# EUROPÄISCHE PATENTANMELDUNG

(11) **EP 4 850 604 A1**
(43) Veröffentlichungstag der Anmeldung: **09.12.2026**
(21) Anmeldenummer: 26182202.7
(22) Anmeldetag: 01.06.2026
(51) Int. Cl.: A61B 5/22, A61B 5/00, A61B 5/11, A63B 23/20

(54) **VORRICHTUNG ZUR DIAGNOSE UND THERAPIE VON BESCHWERDEN DES BECKENBODENS**

(30) Priorität: 03.06.2025 AT 503872025
(71) Anmelder: PelviQueens GmbH, 4224 Wartberg ob der Aist (AT)
(72) Erfinder: Sacher, Judith, 4224 Wartberg ob der Aist (AT); Rechberger, Magdalena, 4224 Wartberg ob der Aist (AT)
(74) Vertreter: KLIMENT & HENHAPEL

(57) **Zusammenfassung**

Vorrichtung zur Diagnose und Therapie von Beschwerden des Beckenbodens mit Sensoren (S1, S2, S3, S4) zur Detektion von Muskelaktivitäten des Beckenbodens, die mit einer Anzeigeeinheit (7) zur Anzeige von Messsignalen der Sensoren (S1, S2, S3, S4) verbunden ist. Es wird vorgeschlagen, dass die Sensoren (S1, S2, S3, S4) als elektromechanische Kraftsensoren ausgeführt sind und ein Kraftaufnehmer für die Sensoren (S1, S2, S3, S4) vorgesehen ist, der als ein entlang einer Längsachse (L) länglicher Formkörper (1) aus einem elastomeren Kunststoff ausgeführt ist, wobei die elektromechanischen Kraftsensoren im Formkörper (1) eingebettet und entlang der Längsachse (L) des Formkörpers (1) verteilt angeordnet sind. Die Vorrichtung ermöglicht im Vergleich zu herkömmlichen Methoden bessere Aussagen über die biomechanische Funktionalität des Beckenbodens.

## Beschreibung

Die Erfindung betrifft eine Vorrichtung zur Diagnose und Therapie von Beschwerden des Beckenbodens mit Sensoren zur Detektion von Muskelaktivitäten des Beckenbodens, die mit einer Anzeigeeinheit zur Anzeige von Messsignalen der Sensoren verbunden ist, gemäß dem Oberbegriff von Anspruch 1.

Der Beckenboden ist der bindegewebs-muskulöse Boden der Beckenhöhle beim Menschen. Er umfasst mehrere Muskeln und Bindegewebsstränge, die in ihrem Zusammenwirken die Beckenhöhle nach unten abschließen und den unteren Teil der Harnröhre sowie die Schließmuskeln der Harnblase und des Anus unterstützen, da Harnröhre, Vagina und Enddarm den Beckenboden queren. Das Anspannen des Beckenbodens sichert die Kontinenz bei Frauen und Männern. Für das kontrollierte Wasserlassen und beim Stuhlgang ist eine gezielte Entspannung von Muskeln des Beckenbodens erforderlich. Schwächungen oder Schädigungen des Beckenbodens können daher zu mangelnder Kontrolle der Ausscheidungsorgane wie Inkontinenz oder ständigem Harndrang führen. Bei Frauen und Männern wird der Beckenboden vor allem durch Übergewicht, chronische körperliche Überlastung oder schlechte Haltung geschwächt. Bei der Frau kann die Beckenbodenmuskulatur durch vaginale Geburten geschwächt oder auch dauerhaft geschädigt werden.

Durch gezieltes Training können Beschwerden aufgrund einer Schwächung oder Schädigung des Beckenbodens erleichtert oder auch beseitigt werden. Dieses Training wird zumeist unter therapeutischer Begleitung durchgeführt und umfasst Übungen, die den Beckenboden stärken und zu einer besseren Kontrolle der Beckenbodenmuskulatur führen. Um die korrekte Ausführung dieser Übungen überprüfen zu können wurden unterschiedliche Vorrichtungen vorgeschlagen, die die Aktivität von Muskeln des Beckenbodens während der Durchführung der Übungen messen. Diese Messungen werden der Patientin oder dem Patienten mitunter auch sofort angezeigt, um eine unmittelbare Reaktion in der Übungsausführung zu ermöglichen. Bei einer solchen Vorgangsweise wird auch von Biofeedback gesprochen.

Hierfür sind unterschiedliche Ansätze bekannt. So wurden etwa transvaginale Biofeedback-Geräte vorgeschlagen, die über eine vaginal eingeführte Sonde die Muskelaktivität im Beckenbodenbereich messen. Das Einführen der Sonde ist für viele Frauen aber unangenehm und für bestimmte Krankheitsbilder, wie beispielsweise Vaginismus, oder in der Zeit nach einer Geburt auch überhaupt nicht möglich. Zudem kann die Anwendung bei Beschwerden wie einer überaktiven Blase, Periodenschmerzen oder Endometriose sogar kontraproduktiv wirken, da diese Beschwerden durch das Einführen verstärkt werden können. Ein weiterer Nachteil besteht in der eingeschränkten Anwendbarkeit der Geräte: Sie können nur in wenigen Positionen, wie Rückenlage, Sitzen oder Stehen, genutzt werden. Bewegungen, wie das Wechseln zwischen diesen Positionen, können die Messungen verfälschen und sind aufgrund der Form und Größe der Sonde oft unangenehm. Bereits leichte Veränderungen der Körperhaltung oder Gewichtsverlagerungen beeinträchtigen die Messergebnisse. Insbesondere sind transvaginale Biofeedback-Geräte nicht für den Einsatz in Bewegung geeignet. Sie funktionieren nur in einer ruhigen Umgebung und erfordern in der Regel keine therapeutische Anleitung, was in einigen Fällen problematisch sein kann. Ein weiterer Nachteil ist, dass der Beckenbodenmuskel die Sonde umschließt, wodurch er nicht in seiner natürlichen, physiologischen Form arbeiten kann.

In der WO 2020/205431 wurde ein Biofeedback-Gerät auf Basis der EMG (Elektromyografie) vorgeschlagen. Bei der EMG wird die elektrische Muskelaktivität anhand von Aktionsströmen der Muskeln gemessen und grafisch als Elektromyogramm dargestellt. Hierfür findet ein sattelförmiger Träger Anwendung, auf dessen Oberfläche etwa 20 Elektroden matrixförmig angeordnet sind. Der Träger selbst wird im Bereich des Perineums angeordnet und kann auch mit Elektroden zur Elektrostimulation kombiniert werden. Bei einer Oberflächen-EMG dieser Art erfassen die Oberflächenelektroden die elektrische Aktivität von Muskeln aufgrund ihrer mechanischen Dimensionen bis zu einigen Zentimetern, sie können aber nicht die Gesamtaktivität größerer Muskeln oder das Zusammenwirken mehrerer Muskeln untereinander oder mit dem umgebenden Bindegewebe abbilden. Wie bereits ausgeführt umfasst der Beckenboden mehrere Muskeln und Bindegewebsstränge, die in ihrer Gesamtheit eine biomechanische Funktionalität ergeben, die über Funktion oder Dysfunktion des Beckenbodens entscheidet. Hier sind vor allem die Muskeln des Musculus levator ani zu nennen, der im wesentlichen aus dem Musculus puborectalis, der eine Schlaufe um das Rektum bildet und somit maßgeblich den Stuhlgang kontrolliert, dem Musculus pubococcygeus, der die Harnröhre gegen das umliegende Gewebe komprimiert und maßgeblich den Harnfluss kontrolliert, und dem Musculus iliococcygeus, der dem Heben und Schließen des Anus dient, gebildet wird. Dieser Musculus levator ani ist einerseits über Bindegewebsstränge mit Knochen des Beckens verbunden und andererseits mit dem Centrum tendineum perinei, das eine faserreiche, sehnige Bindegewebsplatte des Damms (Perineum) ist und den mechanischen Mittelpunkt des muskulären Beckenbodens bildet. In das Centrum tendineum perinei strahlen ferner der Musculus transversus perinei superficialis, der Musculus transversus perinei profundus, der den Hiatus urogenitalis verschließt, durch welche die Harnröhre und die Vagina ziehen, der Musculus sphincter ani externus, der durch einen Dauertonus für den Verschluss des Anus sorgt und mit einer willkürlichen Erschlaffung den Stuhlgang einleitet, und der Musculus bulbospongiosus, der beim Mann der Kompression und Entleerung der Harnröhre dient, ein.

Die Biomechanik des Musculus levator ani in seinem Zusammenwirken mit dem Centrum tendineum perinei kann über vier wesentliche Kräfte beschrieben werden, die bei Kontraktion ventral (bauchwärts) und medial (zur Körpermitte) wirken, und bei Entspannung dorsal (zum Rücken) und lateral (weg von der Körpermitte). Grundsätzlich verschließen die ventral und medial wirkenden Kräfte Körperöffnungen, und die dorsal und lateral wirkenden Kräfte geben Körperöffnungen frei. Die Stärke der Kontraktion hängt allerdings auch von den Bindegewebssträngen des betreffenden Muskels ab. Ist das Bindegewebe geschwächt, kann auch bei hoher Aktivität des Muskels beispielsweise seine Schließfunktion nur unzureichend erfüllt werden. Daher lässt sich durch eine bloße Vermessung der elektrischen Aktivität eines Muskels wie in der WO 2020/205431 die biomechanische Funktionalität des Beckenbodens nur unzureichend beschreiben.

Es besteht daher das Ziel der Erfindung darin eine Vorrichtung zur Diagnose und Therapie von Beckenboden-assoziierten Beschwerden bereitzustellen, die in ihrer Anwendung einfach und für die Patientin oder den Patienten so komfortabel wie möglich ist und Messungen zur Beckenbodenaktivierung auch in Bewegung während der Ausführung von Übungen zur Stärkung des Beckenbodens ermöglicht, wobei die Messung bessere Aussagen über die biomechanische Funktionalität des Beckenbodens ermöglichen soll.

Diese Ziele werden durch die Merkmale von Anspruch 1 erreicht. Anspruch 1 bezieht sich auf eine Vorrichtung zur Diagnose und Therapie von Beschwerden des Beckenbodens mit Sensoren zur Detektion von Muskelaktivitäten des Beckenbodens, die mit einer Anzeigeeinheit zur Anzeige von Messsignalen der Sensoren verbunden ist. Erfindungsgemäß wird vorgeschlagen, dass die Sensoren als elektromechanische Kraftsensoren ausgeführt sind und ein Kraftaufnehmer für die Sensoren vorgesehen ist, der als ein entlang einer Längsachse länglicher Formkörper aus einem elastomeren Kunststoff ausgeführt ist, und die elektromechanischen Kraftsensoren im Formkörper eingebettet sind, wobei mehrere Sensoren entlang der Längsachse des Formkörpers verteilt angeordnet sind, und zur Positionierung des Formkörpers ein um den Unterbauch tragbarer textiler Träger mit einem hüfthohen Bund und einem Schrittbereich vorgesehen ist, wobei eine Aufnahme im Schrittbereich vorgesehen ist, in der der Formkörper aufgenommen und mit Anschlussstellen elektrischer Leitungen zu einem Sendemodul elektrisch kontaktierbar ist, wobei das Sendemodul am textilen Träger vom Formkörper beabstandet angeordnet und mit der Anzeigeeinheit zur drahtlosen Übermittlung und Anzeige der Messsignale der Sensoren des Formkörpers verbunden ist. Der Formkörper weist eine Länge von etwa 10-15cm auf und wird im Zuge der Anwendung im Bereich des Perineums zwischen Steißbein und Schambein am Körper anliegend platziert. Hierfür können eigene Textilien verwendet werden, wie noch näher ausgeführt werden wird. Bei der Durchführung von Übungen zum Trainieren des Beckenbodens wirken Kräfte auf den Formkörper, die durch den elastomeren Kunststoff aufgenommen und an die im Formkörper eingebetteten Sensoren weitergeleitet werden. Elastomere sind bei Raumtemperatur formfeste, aber elastisch verformbare Kunststoffe. Die Kunststoffe können sich bei Zug- und Druckbelastung verformen, finden aber danach wieder in ihre ursprüngliche, unverformte Gestalt zurück. Die Sensoren selbst sind als elektromechanische Kraftsensoren ausgeführt und detektieren somit die auf den jeweiligen Sensor wirkende Kraft durch Bildung einer elektrischen Ladung, die gemessen und angezeigt werden kann. Die erfindungsgemäß vorgesehenen elektromechanischen Kraftsensoren ermöglichen eine direkte Messung der ventral/medialen sowie dorsal/lateralen Öffnungs- und Schließkräfte unterschiedlicher Muskeln vor allem des Musculus levator ani und somit einen genaueren Rückschluss auf die biomechanische Funktionalität des Beckenbodens. Durch die entlang der Längsrichtung des Formkörpers verteilte Anordnung mehrerer Sensoren können dabei unterschiedliche Muskeln und Muskelabschnitte vor allem des Musculus levator ani gemessen werden. Die erfindungsgemäße Vorrichtung muss aber nicht in eine Körperöffnung eingeführt werden, was deren Anwendung für die Patientinnen und Patienten sehr erleichtert. Sie kann somit im Zuge des Beckenbodentrainings auch während der Durchführung von Bewegungen getragen werden. Wie noch genauer ausgeführt werden wird können dabei die Messsignale der Sensoren auf einer Anzeigeeinheit angezeigt werden. Auf diese Weise kann eine visuelle Kontrolle des Trainingsfortschritts ermöglicht werden, die auf der therapeutischen Seite die Trainingsplanung sowie Trainingsüberwachung sehr erleichtert, und auf der Patientenseite die richtige Durchführung von Übungen erleichtert.

Die Sensoren sind vorzugsweise als piezoelektrische Sensoren ausgeführt. Piezoelektrische Sensoren messen unmittelbar die auf den jeweiligen Sensor wirkende Kraft und wandeln sie in eine mit der Kraft korrelierende elektrische Ladung um. Dabei wird vorgeschlagen zumindest zwei Sensoren im Formkörper anzuordnen, wobei einer der Sensoren bei Platzierung der Vorrichtung im Bereich des Perineums im Bereich der Vagina angeordnet ist und ein weiterer Sensor im Bereich des Centrum tendineum perinei, das aufgrund der Vielzahl an einmündenden Muskeln, die in unterschiedliche Richtungen spannen, und als Ankerpunkt vor allem der quergestreiften Muskelfasern einen idealen Messpunkt bildet. Vorzugsweise werden zwei weitere Sensoren entlang der Längsrichtung des Formkörpers angeordnet, von denen einer etwa im Bereich des Anus und ein weiterer etwa im Bereich des Steißbeins angeordnet ist. Gemäß einer weiteren Ausführungsform kann aber auch mit drei Sensoren das Auslangen gefunden werden, wobei der zuvor erwähnte Sensor im Bereich des Steißbeins nicht verwendet wird.

Um einen guten Kontakt zum Messbereich herzustellen wird zudem vorgeschlagen, dass der Formkörper im Bereich von zumindest einem Sensor eine quer zur Längsachse höckerförmig vorragende Oberflächenwölbung aufweist. Diese in Gebrauchslage kranial (nach oben, "scheitelwärts") vorstehenden Oberflächenwölbungen werden vorzugsweise im Bereich jener Sensoren angeordnet, die im Bereich einer Körperöffnung wie der Vagina oder dem Anus liegen, sowie im Dammbereich. Durch die Wölbung werden vor allem im Bereich von Körperöffnungen wie der Vagina oder dem Anus auftretende Kräfte besser vom Formkörper abgenommen.

Um die Kräfte innerhalb des Formkörpers optimal zu einem der eingebetteten Sensoren zu leiten wird zudem vorgeschlagen, dass der Formkörper aus einem elastomeren Kunststoffmaterial der Shore-Härte 40-80 gefertigt ist. Gegebenenfalls kann dieser Formkörper mit einer äußeren Umhüllung aus einem medizinischen Silikon versehen sein. Unter medizinischen Silikonen werden Silikone verstanden, die über eine geprüfte Biokompatibilität verfügen.

Des Weiteren wird vorgeschlagen, dass die Sensoren jeweils streifenförmig ausgeführt und im Formkörper eingebettet sind. Hierfür geeignete piezoelektrische Sensoren sind als piezoelektrische Dünnschichten ausgeführt, beispielsweise aus einem piezoelektrischen PVDF-Polymer, das auf einem Polyester-Substrat aufgebracht ist. Beidseits der piezoelektrischen PVDF-Polymerschicht sind Elektroden aus Silber aufgedruckt, und die gesamte Anordnung über eine Acryl-Beschichtung elektrisch isoliert und geschützt. Solche Dünnschichten zeigen bei Krafteinwirkung und der dadurch bewirkten Auslenkung aus einem mechanisch spannungslosen Zustand eine hohe Dehnungs- und Drucksensitivität. Eine Deformation der piezoelektrischen Elemente durch Druck oder Dehnung erzeugt eine Ladungsverschiebung innerhalb des Sensors, die als direktes Spannungs-/Strom-Messsignal genutzt werden kann.

Zudem wird vorgeschlagen, dass die Sensoren jeweils streifenförmig ausgeführt sind und die Sensoren in ihrer in Längsachsrichtung des Formkörpers verlaufenden Erstreckung parallel zur Längsachse des Formkörpers eingebettet ist. Gemäß einer alternativen Ausführungsform kann aber auch vorgesehen sein, dass die Sensoren jeweils streifenförmig ausgeführt sind und zumindest einer der Sensoren in seiner in Längsachsrichtung des Formkörpers verlaufenden Erstreckung in einem Anstellwinkel zur Längsachse des Formkörpers eingebettet ist. Für eine konkrete Ausführung wird etwa vorgeschlagen, dass ein vorderster, erster Sensor in einem Anstellwinkel von 5-10° zur Längsachse des Formkörpers eingebettet ist, ein zweiter Sensor in seiner in Längsachsrichtung des Formkörpers verlaufenden Erstreckung parallel zur Längsachse eingebettet ist, ein dritter Sensor in einem Anstellwinkel von 15-20° zur Längsachse des Formkörpers eingebettet ist, und ein vierter Sensor in seiner in Längsachsrichtung des Formkörpers verlaufenden Erstreckung parallel zur Längsachse eingebettet ist. Der vorderste, erste Sensor ist jener, der in Gebrauchslage ventral angeordnet ist. Die weitere Reihung über den zweiten bis zum vierten Sensor erfolgt in Gebrauchslage in dorsaler Richtung. Durch die angewinkelte Anordnung des ersten und dritten Sensors relativ zur Längsachse des Formkörpers wird eine für die Kraftmessung vorteilhafte Positionierung des jeweiligen Sensors relativ zu den ihnen jeweils zugeordneten Körperöffnungen der Vagina und des Anus erreicht.

Während der Durchführung entsprechender Übungen zum Trainieren der Beckenbodenmuskulatur muss der Formkörper im Bereich des Perineums auch bei Bewegungen sicher am Körper positioniert sein und die Übertragung von Messsignalen der Sensoren des Formkörpers gewährleistet sein. Hierfür wird erfindungsgemäß vorgeschlagen, dass zur Positionierung des Formkörpers ein um den Unterbauch tragbarer textiler Träger mit einem hüfthohen Bund und einem Schrittbereich vorgesehen ist, wobei eine Aufnahme im Schrittbereich vorgesehen ist, in der der Formkörper aufgenommen und mit Anschlussstellen elektrischer Leitungen zu einem Sendemodul elektrisch kontaktierbar ist, wobei das Sendemodul am textilen Träger vom Formkörper beabstandet angeordnet und mit der Anzeigeeinheit zur drahtlosen Übermittlung und Anzeige der Messsignale der Sensoren des Formkörpers verbunden ist. Bei dem textilen Träger handelt es sich um einen um den Unterbauch tragbaren textilen Träger beispielsweise in Form eines hüfthohen Slips oder "Panties", das aus textilen Rohstoffen wie Naturfasern oder Chemiefasern hergestellt sein kann und hinsichtlich Dehnbarkeit und Hautverträglichkeit einen möglichst hohen Tragekomfort aufweisen sollte. Aus dem Bereich "intelligenter Textilien" (smart textiles) ist es bekannt solche textilen Träger mit elektrischen Leitungen zwischen Sensoren und elektronischen Modulen, die jeweils am textilen Träger angeordnet sind, zu versehen, beispielsweise in Form dünner Drähte oder elektrisch leitfähiger Garne, die in das Textil eingearbeitet sind. Solche textilen Träger sind auch waschbar, wobei im Zuge der vorliegenden Erfindung vorgeschlagen wird, dass der Formkörper in der Aufnahme des textilen Trägers verbleibt. Die Anschlussstellen zu den elektrischen Leitungen können beispielsweise als elektrisch leitende Anschlussflächen ausgeführt sein, die auf einer Innenfläche der Aufnahme angeordnet sind und jeweils an der Oberfläche des Formkörpers vorgesehene Anschlussflächen eines eingeschobenen Formkörpers durch Aneinanderliegen elektrisch kontaktieren. Freilich ist es auch denkbar die elektrischen Anschlusskabel der Sensoren aus dem Formkörper herauszuführen und über Steckverbindungen und dergleichen mit den elektrischen Leitungen des textilen Trägers elektrisch zu kontaktieren. Gegebenenfalls kann im Formkörper auch eine Platine angeordnet sein, die ausgelegt ist die Messsignale der Sensoren zu filtern, zu verstärken und zu digitalisieren und sie danach an das Sendemodul zu senden.

Das Sendemodul empfängt die von den Sensoren gelieferten Messsignale und überträgt sie über eine Nahbereichs-Kommunikationsschnittstelle in Echtzeit an die Anzeigeeinheit. Die Nahbereichs-Kommunikationsschnittstelle ist beispielsweise als Bluetooth-, WLAN-oder UWB-Schnittstelle ausgeführt, über die lediglich in einem begrenzten Radius von bis zu 100 m, gegebenenfalls auch nur bis zu 10m, mit anderen Geräten drahtlose Übertragungskanäle aufgebaut und Daten ausgetauscht werden können. Eine Möglichkeit besteht etwa in der Verwendung einer "Bluetooth Low Energy (BLE)"-Platine im Sendemodul, mit der in einem begrenzten Radius im Bereich weniger Meter mit anderen Geräten Daten ausgetauscht werden können, wobei der Stromverbrauch sehr gering gehalten werden kann. Bei der Anzeigeeinheit kann es sich etwa um ein Mobiltelefon ("Smartphone") handeln, oder auch um ein Tablet, einen Laptop, oder um ein sonstiges mit einem Router oder Gateway versehenes Gerät, das Mobilfunkverbindungen aufbauen und nützen kann. Ein solches Mobilfunkendgerät kann mit einer anwenderspezifischen Bediensoftware (beispielsweise als "Application" oder "App") versehen sein, die von einem Anbieter der erfindungsgemäßen Vorrichtung bezogen werden kann. Das Sendemodul und gegebenenfalls auch der Formkörper sind ferner auch mit einer Stromversorgung beispielsweise in Form eines Akkus versehen, der austauschbar am Textil angeordnet ist.

An der Anzeigeeinheit erfolgt die graphische Aufbereitung und Anzeige der Messsignale über die Bediensoftware ("App"), wobei es therapeutisch angezeigt sein kann die Messsignale mehrerer Sensoren bei bestimmten Übungen oder Therapiestufen in Form eines Summensignals gemeinsam oder auch einzeln anzuzeigen. Dabei kann auch vorgesehen sein die Signale unterschiedlicher Sensoren entweder gleichzeitig anzuzeigen, oder immer nur ein Signal nach Wahl. Die Trainingsplanung, die Durchführung von Übungen zum Trainieren des Beckenbodens, und die Überwachung der Übungsdurchführung mittels der angezeigten Messsignale erfolgt dabei vorzugsweise unter therapeutischer Schulung und Begleitung.

Zudem wird vorgeschlagen, dass der textile Träger mit einer Aufnahmetasche im Bereich des bauchseitig hüfthohen Bundes zur Aufnahme eines weiteren, externen Sensors vorgesehen ist, der in Form eines streifenförmig ausgeführten elektromechanischen Kraftsensors ausgeführt und mit Anschlussstellen weiterer elektrischer Leitungen zum Sendemodul elektrisch kontaktierbar ist. Der externe Sensor kann ebenfalls als piezoelektrischer Sensor ausgeführt sein und erstreckt sich in Gebrauchslage mit einer Länge von etwa 10-15cm horizontal über den Unterleib. Wie die Sensoren des Formkörpers kann auch der externe Sensor als piezoelektrische Dünnschicht beispielsweise in Form eines PVDF-Polymers ausgeführt sein, allerdings mit größeren Abmessungen. Die Verwendung des externen Sensors ermöglicht zusätzliche therapeutische und diagnostische Möglichkeiten. Der im Bereich des bauchseitig hüfthohen Bundes angeordnete Sensor kommt im Bereich des Musculus transversus abdominis zu liegen, der zwar außerhalb des Beckenbodens liegt, aber ein Synergist der Beckenbodenmuskulatur ist. Er kontrahiert gemeinsam mit der Beckenbodenmuskulatur und optimiert dabei die Funktion der Beckenbodenmuskulatur. Im Zuge des Trainierens der Beckenbodenmuskulatur kann es daher vorteilhaft sein auch die Tätigkeit des Musculus transversus abdominis zu überwachen und in das Training einzubeziehen. So kann es etwa bei Beginn einer Therapie zur Stärkung einer stark geschwächten Beckenbodenmuskulatur vorteilhaft sein das Anspannen des Musculus transversus abdominis bewusst zu vermeiden, was durch Beobachtung des entsprechenden Messsignals überwacht werden kann. Erst nach einem gewissen Trainingsfortschritt kann unter therapeutischer Anleitung mit einem koordinierten Training des Musculus transversus abdominis begonnen werden. Diese Trainingsplanung erfolgt durch therapeutisch entsprechend geschultes Personal, das vor Trainingsbeginn zunächst die Grundspannung der Beckenbodenmuskulatur ermitteln wird, um danach ein darauf abgestimmtes Therapieprogramm zu erstellen.

Die Erfindung wird in weiterer Folge anhand von Ausführungsbeispielen mithilfe der beiliegenden Figuren näher erläutert. Es zeigen hierbei die
Fig. 1 eine Ausführungsform eines erfindungsgemäßen Formkörpers gemäß der Fig. 2 von oben gesehen mit den eingezeichneten Schnittebenen A-A und B-B,
Fig. 2 eine Seitenansicht einer Ausführungsform eines erfindungsgemäßen Formkörpers mit den eingezeichneten Lagen der Sensoren des Formkörpers,
Fig. 3 eine Schnittansicht entlang der Schnittebene A-A gemäß der Fig. 1,
Fig. 4a eine Schnittansicht entlang der Schnittebene B-B gemäß der Fig. 1 für eine erste Ausführungsform des Formkörpers mit Sensoren, die in einem Anstellwinkel zur Längsachse des Formkörpers angeordnet sind,
Fig. 4b eine Schnittansicht entlang der Schnittebene B-B gemäß der Fig. 1 für eine zweite Ausführungsform des Formkörpers mit flacher Anordnung der Sensoren,
Fig. 5 eine schematische Ansicht zur Erläuterung einer Anwendung der erfindungsgemäßen Vorrichtung,
Fig. 6 einen ersten Anzeigemodus an der Anzeigeeinheit, bei dem ein Summensignal der Messsignale der Sensoren für die Beckenbodenmuskulatur angezeigt wird, sowie getrennt dazu das Messsignal des externen Sensors am Musculus transversus abdominis, und die
Fig. 7 einen zweiten Anzeigemodus an der Anzeigeeinheit, bei dem die Messsignale der Sensoren für die Beckenbodenmuskulatur jeweils getrennt voneinander angezeigt werden, sowie getrennt dazu das Messsignal des externen Sensors am Musculus transversus abdominis.

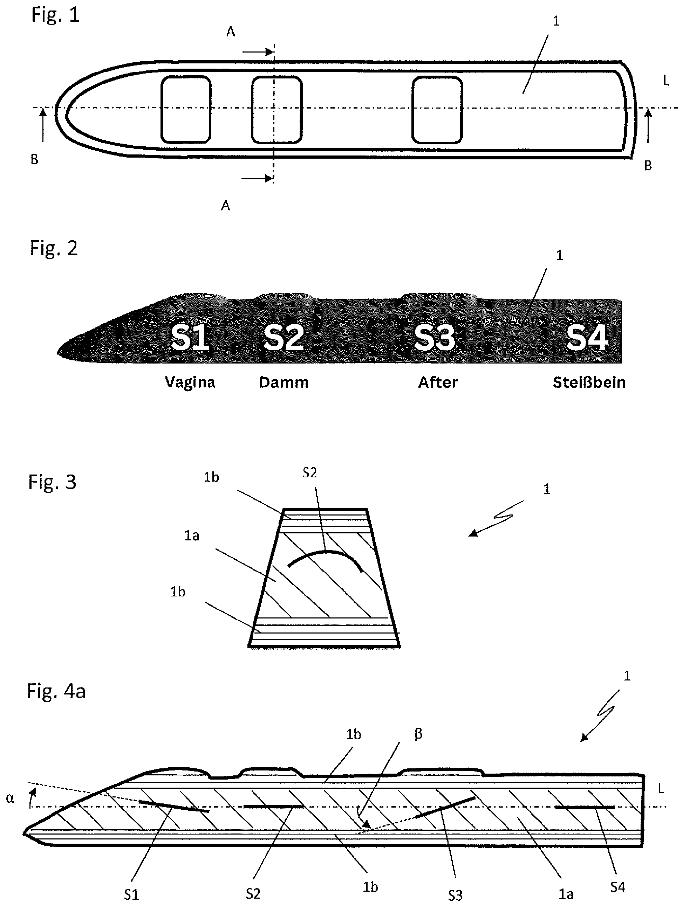

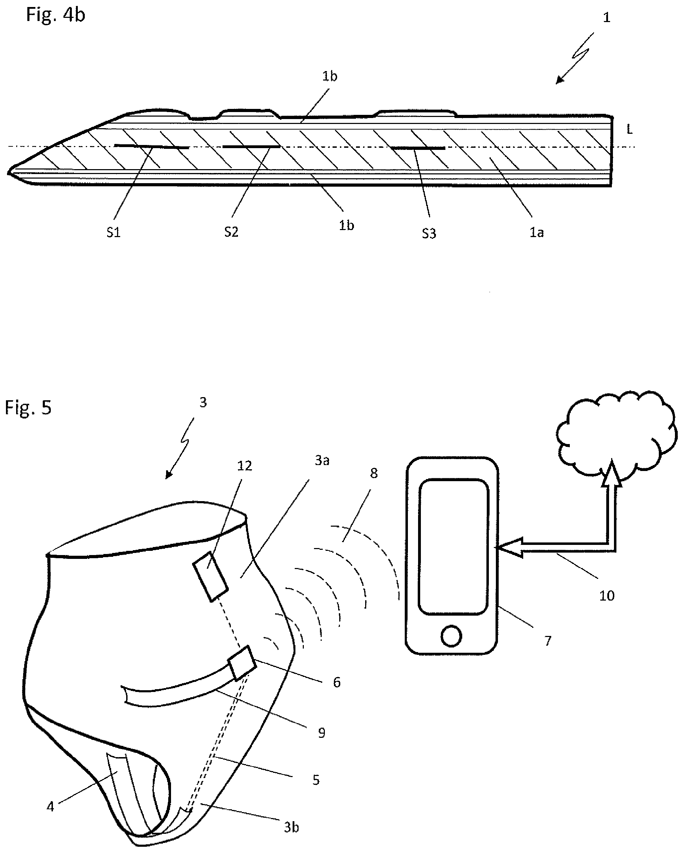

Zunächst wird auf die Fig. 1-4 Bezug genommen, die Ausführungsformen des erfindungsgemäßen Formkörpers 1 zeigen. Der Formkörper 1 ist als länglicher Formkörper 1 aus einem elastomeren Kunststoff mit der Längsachse L ausgeführt. Der Formkörper 1 dient als Kraftaufnehmer für Sensoren S1, S2, S3, S4, die jeweils als elektromechanische Kraftsensoren ausgeführt sind und entlang der Längsachse L des Formkörpers 1 im Formkörper 1 eingebettet verteilt angeordnet sind. Der Formkörper 1 weist eine Länge von etwa 10-15cm auf, sodass die Darstellungen der Fig. 2 und 4 in etwa maßstabsgetreu sind, und wird im Zuge der Anwendung im Bereich des Perineums zwischen Steißbein und Schambein am Körper anliegend platziert, wie noch näher ausgeführt werden wird. Für das Kunststoffmaterial des Formkörpers 1 kann beispielsweise Silikon der Shore-Härte 40-80 verwendet werden.

Um einen guten Kontakt zum Messbereich herzustellen weist der Formkörper 1 zudem im Bereich der Sensoren S1, S2, S3 quer zur Längsachse L höckerförmig vorragende Oberflächenwölbungen 2 auf. Diese Oberflächenwölbungen 2 werden vorzugsweise im Bereich jener Sensoren S1, S2, S3 angeordnet, die im Bereich einer Körperöffnung wie der Vagina oder dem Anus liegen, sowie im Dammbereich. In der Fig. 2 ist ersichtlich, dass der vorderste, ventrale Sensor S1 bei Gebrauch der Vorrichtung im Bereich der Vagina liegt, der in dorsaler Richtung benachbarte Sensor S2 im Bereich des Centrum tendineum perinei im Dammbereich, und der in dorsaler Richtung folgende Sensor S3 im Bereich des Anus. In diesen Bereichen ist der Formkörper 1 mit den in Gebrauchslage kranial (in Kopfrichtung, "nach oben") vorstehenden Oberflächenwölbungen 2 versehen. Bei dem in dorsaler Richtung letzten Sensor S4, der bei Gebrauch der Vorrichtung im Bereich des Steißbeins liegt, ist keine solche Oberflächenwölbung 2 vorgesehen. Durch die Oberflächenwölbungen 2 werden vor allem im Bereich von Körperöffnungen wie der Vagina oder dem Anus auftretende Kräfte besser vom Formkörper 1 abgenommen. Die beiden Enden des länglichen Formkörpers 1 verjüngen sich in Längsrichtung, sodass der Querschnitt des Formkörpers 1 senkrecht zur Längsachse L an den beiden Enden nach außen zu immer kleiner wird. Zudem ist der Formkörper 1 mit Einschnitten 11 versehen, die eine bessere Biegefähigkeit des Formkörpers 1 bewirken und den Tragekomfort somit verbessern.

Die Sensoren S1, S2, S3, S4 sind als piezoelektrische Sensoren S1, S2, S3, S4 ausgeführt. Die piezoelektrische Sensoren S1, S2, S3, S4 messen unmittelbar die auf den jeweiligen Sensor S1, S2, S3, S4 wirkende Kraft und wandeln sie in eine mit der Kraft korrelierende elektrische Ladung um. Hierfür sind die Sensoren S1, S2, S3, S4 jeweils streifenförmig ausgeführt und im Formkörper 1 eingebettet. Hierfür geeignete piezoelektrische Sensoren S1, S2, S3, S4 sind als piezoelektrische Dünnschichten ausgeführt, beispielsweise aus einem piezoelektrischen PVDF-Polymer, das auf einem Polyester-Substrat aufgebracht ist. Beidseits der piezoelektrischen PVDF-Polymerschicht sind Elektroden aus Silber aufgedruckt, und die gesamte Anordnung über eine Acryl-Beschichtung elektrisch isoliert und geschützt. Eine Deformation der piezoelektrischen Elemente durch Druck oder Dehnung erzeugt eine Ladungsverschiebung innerhalb der Sensoren S1, S2, S3, S4, die als direktes Spannungs-/Strom-Messsignal genutzt werden kann.

Wie der Fig. 4a entnommen werden kann, sind die Sensoren S1 und S3 zudem in ihrer in Längsachsrichtung des Formkörpers 1 verlaufenden Erstreckung in einem Anstellwinkel α, β zur Längsachse L des Formkörpers 1 eingebettet. Ein vorderster, erster Sensor S1 ist dabei in einem Anstellwinkel β von 5-10° zur Längsachse L des Formkörpers 1 eingebettet, und ein weiterer Sensor S3 in einem Anstellwinkel α von 15-20° zur Längsachse L des Formkörpers 1, allerdings im Vergleich zum Anstellwinkel β des ersten Sensors S1 im entgegengesetzten Uhrzeigersinn gemessen, wie der Fig. 4a entnommen werden kann. Die verbleibenden Sensoren S2 und S4 sind in ihrer in Längsachsrichtung des Formkörpers 1 verlaufenden Erstreckung parallel zur Längsachse L eingebettet. Durch die in einem Anstellwinkel α, β erfolgte Anordnung des ersten Sensors S1 und des dritten Sensors S3 zur Längsachse L des Formkörpers 1 wird eine für die Kraftmessung vorteilhafte Positionierung des jeweiligen Sensors S1, S3 relativ zu den ihnen jeweils zugeordneten Körperöffnungen der Vagina und des Anus erreicht.

In der Fig. 4b ist eine Schnittansicht entlang der Schnittebene B-B gemäß der Fig. 1 für eine zweite Ausführungsform des Formkörpers 1 mit insgesamt drei Sensoren S1, S2, S3 in jeweils flacher Anordnung der Sensoren S1, S2, S3 gezeigt, bei der die Sensoren S1, S2, S3 in ihrer in Längsachsrichtung des Formkörpers 1 verlaufenden Erstreckung im Wesentlichen parallel zur Längsachse L des Formkörpers 1 eingebettet sind.

Bei der Durchführung von Übungen zum Trainieren des Beckenbodens wirken Kräfte auf den Formkörper 1, die durch den elastomeren Kunststoff aufgenommen und an die im Formkörper 1 eingebetteten Sensoren S1, S2, S3, S4 weitergeleitet werden. Die Sensoren ermöglichen als elektromechanische Kraftsensoren eine direkte Messung der ventral/medialen sowie dorsal/lateralen Öffnungs- und Schließkräfte unterschiedlicher Muskeln vor allem des Musculus levator ani und somit einen genaueren Rückschluss auf die biomechanische Funktionalität des Beckenbodens. Durch die entlang der Längsachse L des Formkörpers 1 verteilte Anordnung mehrerer Sensoren S1, S2, S3, S4 können dabei unterschiedliche Muskeln und Muskelabschnitte vor allem des Musculus levator ani gemessen werden.

Eine mögliche Anwendung des Formkörpers 1 im Rahmen der erfindungsgemäßen Vorrichtung wird in weiterer Folge anhand der Fig. 5-7 erläutert. Während der Durchführung entsprechender Übungen zum Trainieren der Beckenbodenmuskulatur muss der Formkörper 1 im Bereich des Perineums auch bei Bewegungen sicher am Körper positioniert sein und die Übertragung von Messsignalen der Sensoren S1, S2, S3, S4 des Formkörpers 1 gewährleistet sein. Hierfür ist ein um den Unterbauch tragbarer textiler Träger 3 beispielsweise in Form eines hüfthohen Slips oder "Pantie" mit einem hüfthohen Bund 3a und einem Schrittbereich 3b vorgesehen. Zur Positionierung des Formkörpers 1 ist im Schrittbereich 3b eine Aufnahme 4 eingearbeitet, in der der Formkörper 1 aufgenommen und fixiert wird. Bei der Positionierung des Formkörpers 1 in der Aufnahme 4 ist er zudem mit Anschlussstellen elektrischer Leitungen 5 kontaktierbar, über die die Messsignale des Formkörpers 1 an ein Sendemodul 6 übertragen werden. Zur elektrischen Kontaktierung ist es etwa möglich die elektrischen Anschlusskabel der Sensoren S1, S2, S3, S4 aus dem Formkörper 1 herauszuführen und über Steckverbindungen und dergleichen mit den elektrischen Leitungen 5 des textilen Trägers 3 elektrisch zu kontaktieren. Alternativ ist es auch denkbar die elektrischen Anschlusskabel der Sensoren S1, S2, S3, S4 in Anschlussflächen etwa auf einer in Gebrauchslage kaudalen Oberfläche des Formkörpers 1 münden zu lassen, die beim Einlegen des Formkörpers 1 in die Aufnahme 4 in Anlage mit entsprechenden Anschlussflächen auf der Innenfläche der Aufnahme 4 gelangen und bei dieser Anlage einen elektrischen Kontakt herstellen. Die Anschlussflächen können dabei auch als metallische Druckknöpfe oder dergleichen ausgeführt sein, um die Verbindungserstellung zu verbessern. Der Formkörper 1 ist zudem mit einer Platine versehen, mit der die Messsignale der Sensoren S1, S2, S3 und S4 gefiltert, verstärkt und über einen A/D-Wandler digitalisiert werden, bevor sie über die elektrischen Leitungen 5 an das Sendemodul 6 übermittelt werden.

Die elektrischen Leitungen 5 sind beispielsweise in Form dünner Drähte oder elektrisch leitfähiger Garne ausgeführt, die im textilen Träger 3 eingearbeitet sind und eine Weiterleitung der Messsignale entlang des textilen Trägers 3 ermöglichen. Solche Textilien mit elektrischen Verbindungen zwischen am Textil angeordneten Sensorelementen und elektronischen Modulen sind aus dem Bereich "intelligenter Textilien" (smart textiles) hinlänglich bekannt.

Wie insbesondere in der Fig. 5 ersichtlich ist, weist der textile Träger 3 eine weitere Aufnahmetasche 9 im Bereich des bauchseitig hüfthohen Bundes 3a zur Aufnahme eines weiteren, externen Sensors S5 auf. Dieser externe Sensor S5 ist in Form eines streifenförmig ausgeführten piezoelektrischen Sensors S5 ausgeführt und mit einem Mikroprozessor verbunden, der auch das Sendemodul 6 trägt, an den die Messsignale des externen Sensors S5 übertragen werden. Der im Bereich des bauchseitig hüfthohen Bundes 3a angeordnete Sensor S5 kommt im Bereich des Musculus transversus abdominis zu liegen, der zwar außerhalb des Beckenbodens liegt, aber ein Synergist der Beckenbodenmuskulatur ist.

Das Sendemodul 6 ist am textilen Träger 3 vom Formkörper 1 beabstandet angeordnet und empfängt die von den Sensoren S1, S2, S3, S4 sowie vom externen Sensor S5 über die elektrischen Leitungen 5 gelieferten Messsignale und überträgt sie über eine Nahbereichs-Kommunikationsschnittstelle in Echtzeit an die Anzeigeeinheit 7. Die Nahbereichs-Kommunikationsschnittstelle ist beispielsweise als Bluetooth-, WLAN-oder UWB-Schnittstelle ausgeführt, über die lediglich in einem begrenzten Radius von bis zu 100 m, gegebenenfalls auch nur bis zu 10m, mit anderen Geräten drahtlose Übertragungskanäle 8 aufgebaut und Daten ausgetauscht werden können. Eine Möglichkeit besteht etwa in der Verwendung einer "Bluetooth Low Energy (BLE) "-Platine im Sendemodul 6, mit der in einem begrenzten Radius im Bereich weniger Meter mit anderen Geräten Daten ausgetauscht werden können, wobei der Stromverbrauch sehr gering gehalten werden kann. Bei der Anzeigeeinheit 7 kann es sich etwa um ein Mobiltelefon ("Smartphone") handeln, oder auch um ein Tablet, einen Laptop, oder um ein sonstiges mit einem Router oder Gateway versehenes Gerät, das Mobilfunkverbindungen aufbauen und nützen kann. Ein solches Mobilfunkendgerät kann mit einer anwenderspezifischen Bediensoftware (beispielsweise als "Application" oder "App") versehen sein, die von einem Anbieter der erfindungsgemäßen Vorrichtung bezogen werden kann. Das Sendemodul 6 ist ferner auch mit einer Stromversorgung beispielsweise in Form eines Akkus 12 versehen, der austauschbar am textilen Träger 3 in einer eigenen Akku-Aufnahme gehalten ist. Der Akkus 12 versorgt gegebenenfalls auch den Formkörper 1 mit Strom.

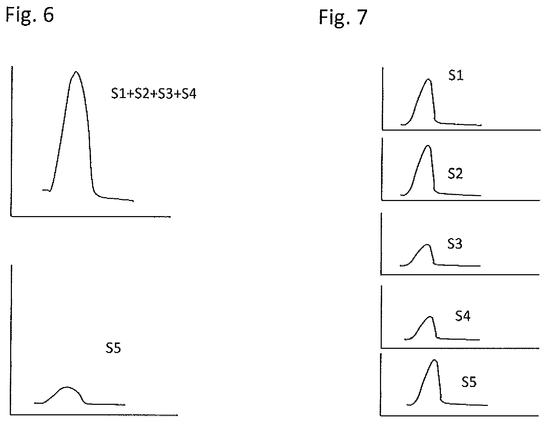

An der Anzeigeeinheit 7 erfolgt die graphische Aufbereitung und Anzeige der Messsignale über die Bediensoftware ("App"), wobei es therapeutisch angezeigt sein kann die Messsignale mehrerer Sensoren S1, S2, S3, S4, S5 bei bestimmten Übungen oder Therapiestufen in Form eines Summensignals gemeinsam oder auch einzeln anzuzeigen, wie in den Fig. 6 und 7 erläutert werden soll. Im oberen Diagramm der Fig. 6 ist etwa zu sehen, wie in einem ersten Anzeigemodus die Beckenboden-bezogenen Signale der Sensoren S1, S2, S3, S4 des Formkörpers 1 als Summensignal angezeigt werden, und gleichzeitig aber getrennt davon das im unteren Diagramm gezeigte Messsignal des externen Sensors S5, das sich auf die Aktivität des Musculus transversus abdominis bezieht. In der Fig. 7 ist hingegen ein zweiter Anzeigemodus dargestellt, bei dem die Messsignale der Sensoren S1, S2, S3, S4 für die Beckenbodenmuskulatur in den oberen vier Diagrammen der Fig. 7 jeweils getrennt angezeigt werden, sowie getrennt dazu das Messsignal des externen Sensors S5 am Musculus transversus abdominis im untersten Diagramm der Fig. 7. Die Signale werden in den Fig. 6 und 7 jeweils über die Zeit aufgetragen. Der erste Anzeigemodus gemäß der Fig. 6 kann etwa bei Beginn einer Therapie zur Stärkung einer stark geschwächten Beckenbodenmuskulatur vorteilhaft sein. In dieser Therapiestufe kann etwa vorgesehen sein beim Anspannen der Beckenbodenmuskulatur das Anspannen des Musculus transversus abdominis bewusst zu vermeiden, was durch Beobachtung des Summensignals im oberen Diagramm der Fig. 6 und des externen Sensors S5 im unteren Diagramm der Fig. 6 überwacht werden kann. Erst nach einem gewissen Trainingsfortschritt kann unter therapeutischer Anleitung mit einem koordinierten Training des Musculus transversus abdominis und einem differenzierten Training der unterschiedlichen Muskeln des Beckenbodens begonnen werden. Hierfür ist der zweite Anzeigemodus gemäß der Fig. 7 vorteilhaft, bei dem die Messsignale der auf den Beckenboden bezogenen Sensoren S1, S2, S3, S4 gemäß den oberen vier Diagrammen der Fig. 7 getrennt voneinander beobachtet werden können, sowie gleichzeitig aber ebenfalls getrennt das Messsignal des externen Sensors S5 im untersten Diagramm der Fig. 7. Dabei kann auch vorgesehen sein über die Bediensoftware einzelne Diagramme auszublenden oder zur exklusiven Darstellung auszuwählen.

Die Trainingsplanung, die Durchführung von Übungen zum Trainieren des Beckenbodens, und die Überwachung der Übungsdurchführung mittels der angezeigten Messsignale erfolgt wie erwähnt vorzugsweise unter therapeutischer Schulung und Begleitung. Hierfür kann auch vorgesehen sein, dass Daten von der Anzeigeeinheit 7 über eine Anbindung 10 an ein weltweites Kommunikationsnetzwerk wie das Internet ausgetauscht werden können, um der Therapeutin oder dem Therapeuten eine Übungsbegleitung, mitunter auch in Echtzeit, zu ermöglichen.

Die Erfindung ermöglicht somit auf einfache und für die Patientin oder den Patienten komfortable Weise die Diagnose und Therapie von Beckenboden-assoziierten Beschwerden. Dabei ist es auch möglich Messungen zur Beckenbodenaktivierung in Bewegung während der Ausführung von Übungen zur Stärkung des Beckenbodens durchzuführen, wobei die Messung im Vergleich zu herkömmlichen Methoden bessere Aussagen über die biomechanische Funktionalität des Beckenbodens ermöglicht.

## Patentansprüche

1. Vorrichtung zur Diagnose und Therapie von Beschwerden des Beckenbodens mit Sensoren (S1, S2, S3, S4) zur Detektion von Muskelaktivitäten des Beckenbodens, die mit einer Anzeigeeinheit (7) zur Anzeige von Messsignalen der Sensoren (S1, S2, S3, S4) verbunden ist, **dadurch gekennzeichnet, dass** die Sensoren (S1, S2, S3, S4) als elektromechanische Kraftsensoren ausgeführt sind und ein Kraftaufnehmer für die Sensoren (S1, S2, S3, S4) vorgesehen ist, der als ein entlang einer Längsachse (L) länglicher Formkörper (1) aus einem elastomeren Kunststoff ausgeführt ist, und die elektromechanischen Kraftsensoren im Formkörper (1) eingebettet sind, wobei mehrere Sensoren (S1, S2, S3, S4) entlang der Längsachse (L) des Formkörpers (1) verteilt angeordnet sind, und zur Positionierung des Formkörpers (1) ein um den Unterbauch tragbarer textiler Träger (3) mit einem hüfthohen Bund (3a) und einem Schrittbereich (3b) vorgesehen ist, wobei eine Aufnahme (4) im Schrittbereich (3b) vorgesehen ist, in der der Formkörper (1) aufgenommen und mit Anschlussstellen elektrischer Leitungen (5) zu einem Sendemodul (6) elektrisch kontaktierbar ist, wobei das Sendemodul (6) am textilen Träger (3) vom Formkörper (1) beabstandet angeordnet und mit der Anzeigeeinheit (7) zur drahtlosen Übermittlung und Anzeige der Messsignale der Sensoren (S1, S2, S3, S4) des Formkörpers (1) verbunden ist.

2. Vorrichtung nach Anspruch 1, **dadurch gekennzeichnet, dass** die Sensoren (S1, S2, S3, S4) als piezoelektrische Sensoren ausgeführt sind.

3. Vorrichtung nach Anspruch 1 oder 2, **dadurch gekennzeichnet, dass** zwei bis vier Sensoren (S1, S2, S3, S4) entlang der Längsachse (L) des Formkörpers (1) verteilt angeordnet sind.

4. Vorrichtung nach einem der Ansprüche 1 bis 3, **dadurch gekennzeichnet, dass** der Formkörper (1) im Bereich von zumindest einem Sensor (S1, S2, S3, S4) eine quer zur Längsachse (L) höckerförmig vorragende Oberflächenwölbung (2) aufweist.

5. Vorrichtung nach einem der Ansprüche 1 bis 4, **dadurch gekennzeichnet, dass** der Formkörper (1) aus einem elastomeren Kunststoffmaterial der Shore-Härte 40-80 gefertigt ist.

6. Vorrichtung nach einem der Ansprüche 1 bis 5, **dadurch gekennzeichnet, dass** die Sensoren (S1, S2, S3, S4) jeweils streifenförmig ausgeführt und im Formkörper (1) eingebettet sind.

7. Vorrichtung nach Anspruch 6, **dadurch gekennzeichnet, dass** drei Sensoren (S1, S2, S3) entlang der Längsachse (L) des Formkörpers (1) verteilt angeordnet sind, und die Sensoren (S1, S2, S3) in ihrer in Längsachsrichtung des Formkörpers (1) verlaufenden Erstreckung jeweils parallel zur Längsachse (L) des Formkörpers (1) eingebettet sind.

8. Vorrichtung nach einem der Ansprüche 1 bis 6, **dadurch gekennzeichnet, dass** die Sensoren (S1, S2, S3, S4) jeweils streifenförmig ausgeführt sind und zumindest einer der Sensoren (S1, S2, S3, S4) in seiner in Längsachsrichtung des Formkörpers (1) verlaufenden Erstreckung in einem Anstellwinkel (α, β) zur Längsachse (L) des Formkörpers (1) eingebettet ist.

9. Vorrichtung nach Anspruch 8, **dadurch gekennzeichnet, dass** ein vorderster, erster Sensor (S1) in einem Anstellwinkel (β) von 5-10° zur Längsachse (L) des Formkörpers (1) eingebettet ist, ein zweiter Sensor (S2) in seiner in Längsachsrichtung des Formkörpers (1) verlaufenden Erstreckung parallel zur Längsachse (L) eingebettet ist, ein dritter Sensor (S3) in einem Anstellwinkel (α) von 15-20° zur Längsachse (L) des Formkörpers (1) eingebettet ist, und ein vierter Sensor (S4) in seiner in Längsachsrichtung des Formkörpers (1) verlaufenden Erstreckung parallel zur Längsachse (L) eingebettet ist.

10. Vorrichtung nach einem der Ansprüche 1 bis 9, **dadurch gekennzeichnet, dass** der textile Träger (3) mit einer Aufnahmetasche (9) im Bereich des bauchseitig hüfthohen Bundes (3a) zur Aufnahme eines weiteren, externen Sensors (S5) vorgesehen ist, der in Form eines streifenförmig ausgeführten elektromechanischen Kraftsensors ausgeführt und mit Anschlussstellen weiterer elektrischer Leitungen (5) zum Sendemodul (6) elektrisch kontaktierbar ist.
